# EUROPEAN PATENT SPECIFICATION

(11) **EP 2 420 282 B1**
(45) Date of publication and mention of the grant of the patent: **18.09.2019**
(21) Application number: 10764451.0
(22) Date of filing: 13.04.2010
(51) Int. Cl.: A61M 25/01, A61M 25/09, A61M 25/00

(54) **MEDICAL GUIDE WIRE**
MEDIZINISCHER FÜHRUNGSDRAHT
FIL GUIDE MÉDICAL

(30) Priority: 14.04.2009 JP 2009098463
(43) Date of publication of application: 22.02.2012
(73) Proprietor: Terumo Kabushiki Kaisha, Tokyo 151-0072 (JP)
(72) Inventor: KUROSAWA, Tomokane, Fujinomiya-shi Shizuoka 418-0015 (JP); TANO, Yutaka, Fujinomiya-shi Shizuoka 418-0015 (JP)
(74) Representative: TBK
(86) International application number: PCT/JP2010/056600
(87) International publication number: WO 2010/119867

(56) References cited:
- EP-A1- 1 920 795
- WO-A1-03/089039
- JP-A- 8 252 262
- JP-A- 2004 181 184
- JP-A- 2004 222 880
- JP-A- 2006 141 778
- JP-A- 2006 230 482
- JP-A- 2007 061 181
- JP-A- 2008 125 628
- JP-A- 2008 125 628
- US-A- 5 295 493
- US-B1- 6 475 195
- US-B1- 6 558 368

## Description

### TECHNICAL FIELD

The present invention relates to a guide wire for medical treatment used for introducing a medical device such as a catheter and the like, which is used by aiming a vascular treatment or checkup, until a desired region inside a blood vessel.

### BACKGROUND ART

A guide wire for medical treatment is used for introducing and dwelling a medical tool such as a catheter and an introducer kit inside a blood vessel when carrying out diagnosis and treatment of a blood vessel percutaneously. In the past, it was a mainstream that a region on an occasion of introducing a medical device such as a catheter into a blood vessel is selected to be a femoral region (FEMORAL-REGION), but in order to reduce burden with respect to a patient, there is a trend, in recent years, of a process of a shift to a brachial region (BRACHIAL-REGION) and, in particular, to a radial region (RADIAL-REGION), and there has been desired a guide wire for medical treatment which can be used safely inside an arm blood vessel that often includes a branch and/or a meander and also, which is excellent in operability.

In the past, there has been used a guide wire for medical treatment having a J-shape at the distal end thereof when carrying it from a radial region (RADIAL-REGION) to a target region in the vicinity of the heart. In this case, insertion is carried out into the blood vessel by setting the J-shape of the distal end upright, but when the guide wire is further pushed ahead in a state in which the J-shape of the distal end reaches a side-branch of the blood vessel, there was such a problem in which it happens that the guide wire is deviated from the aimed blood vessel and is pushed-ahead toward the side-branch. In this case, the operation was complicated such that the operator must pull out the guide wire from the side-branch once and thereafter, must push it forward again by aiming the objective blood vessel. Further, whether or not the guide wire goes erroneously into the side-branch is confirmed usually by using an X-ray contrast and the operator has to inject the contrast agent to the patient in each case thereof, so that there was a fear of physical influence with respect to the patient.

In order to solve the problem mentioned above, there is disclosed in Patent Document 1 about a guide wire which is prevented from going erroneously into a branch of a blood vessel by setting the angle formed between an extended line of a distal straight line portion of a J-shaped guide wire and a wire base line to be 40 degree to 70 degree.

However, in case of carrying a guide wire from a radial region (RADIAL-REGION) to a heart, the guide wire must be advanced through a blood vessel path having a lot of branched blood vessels compared with a femoral region (FEMORAL-REGION) or a brachial region (BRACHIAL-REGION) and even if there is employed a J-shaped guide wire whose distal end is formed by a certain angle, it does not reach a result of solving a problem in which the guide wire goes erroneously into a branched blood vessel, and there has been required a guide wire which can be carried reliably until a target region in the vicinity of the heart. Moreover, even if there is a case in which the J-shape of the distal end goes straight ahead after returning to the original J-shape caused by inversion in a state of getting into the branched blood vessel, there might occur a case in which it happens that the guide wire will go erroneously into a different branched blood vessel depending on the distal shape thereof.

In the past, there has also been used a guide wire for medical treatment as disclosed Patent Document 2. This guide wire has the features set forth in the preamble of claim 1, and the distal portion thereof crosses the proximal section at an acute angle.

### Related Prior Technical Documents

### Patent Documents

Patent Document 1: JP 2004 181184 A
Patent Document 2: JP 2008 125628 A

### DISCLOSURE OF THE INVENTION

As a result of devoting themselves to investigations again and again in order to solve the problem mentioned above, the inventors reached a situation of completing the present invention in which it is possible to improve operability and safety, and also, it is possible to soften a burden of an operator. A guide wire for medical treatment of the present invention for achieving the problem mentioned above is a guide wire for medical treatment including the features set forth in claim 1.

The guide wire for medical treatment of the present invention constituted as mentioned above becomes in a state of having a three dimensional structure in which the distal portion thereof is extended in a direction backing away from the curve plane, so that it is possible to be formed in a spiral shape when the distal portion thereof is extended and it is possible to carry out a guide wire operation in which it is difficult to enter a branched blood vessel caused by a driving force transmission mechanism specific to the spiral shape thereof. Also, even in a case in which the distal portion of the guide wire is hooked in the vicinity of an entrance of a branched blood vessel, it is possible to distribute the pushing force which is added continuously from the hand side, and moreover, it becomes easy for the distal portion of the guide wire to be disengage from the branched blood vessel and it is possible to decrease phenomena of going erroneously into branched blood vessels.

Also, even in a case in which the most distal portion of the guide wire cannot be disengaged from the branched blood vessel, it is possible for the guide wire to return to the shape before extending the distal portion inside the blood vessel (original shape) caused by a further pushing, so that it is possible to restore a safe shape which does not injure the blood vessel wall. Further, even if pushing the guide wire forward in a state of becoming an original shape, it becomes possible for the guide wire to go straight ahead without going into the branched blood vessel.

Also, the most distal portion thereof is provided in a section in a direction, toward which the curved portion is curved, with respect to an orthogonal plane on an axis core of the proximal section orthogonal to the curve plane, so that after the guide wire returns to the original shape inside the blood vessel, it is possible for the guide wire to return to the spiral shape easily by pulling back the guide wire backward after hooking the most distal portion of the guide wire onto the entrance of the branched blood vessel.

### BRIEF DESCRIPTION OF DRAWINGS

FIGS. 1 show a guide wire for medical treatment relating to an exemplified embodiment of the present invention, in which FIG. 1A shows a plan view and FIG. 1B shows a side view;
FIG. 2 is a side view of seeing a guide wire for medical treatment relating to an exemplified embodiment of the present invention from the distal side thereof;
FIG. 3 is a cross-sectional view showing a guide wire for medical treatment inserted into a blood vessel inside;
FIG. 4 is a cross-sectional view showing an occasion when the distal portion of the guide wire for medical treatment inserted into the blood vessel inside will be inverted;
FIG. 5 is a cross-sectional view showing an occasion when the distal portion of the guide wire for medical treatment inserted into the blood vessel inside returns to the original shape;
FIG. 6 is a cross-sectional view showing an occasion when returning the guide wire for medical treatment, which returned to the original shape inside the blood vessel, to a spiral shape;
FIG. 7 is a side view showing a modified example of the distal portion of the guide wire for medical treatment relating to an exemplified embodiment of the present invention;
FIG. 8 is a side view showing another modified example of the distal portion of the guide wire for medical treatment relating to an exemplified embodiment of the present invention;
FIG. 9 is a cross-sectional view showing an occasion when inserting the guide wire for medical treatment shown in FIG. 8 into a blood vessel inside;
FIG. 10 is a side view seeing the modified example of the curved portion of the guide wire for medical treatment relating to the exemplified embodiment of the present invention from the distal side thereof;
FIG. 11 is a side view seeing another modified example of the curved portion of the guide wire for medical treatment relating to the exemplified embodiment of the present invention from the distal side thereof; and
FIG. 12 is a plan view showing another modified example of the distal portion of the guide wire for medical treatment relating to the exemplified embodiment of the present invention.

### BEST MODE FOR CARRYING OUT THE INVENTION

Hereinafter, it will be explained with respect to exemplified embodiments of the present invention with reference to the drawings. It should be noted that there is a case in which the size ratio in the drawings is exaggerated for convenience of explanation and is different from the actual ratio.

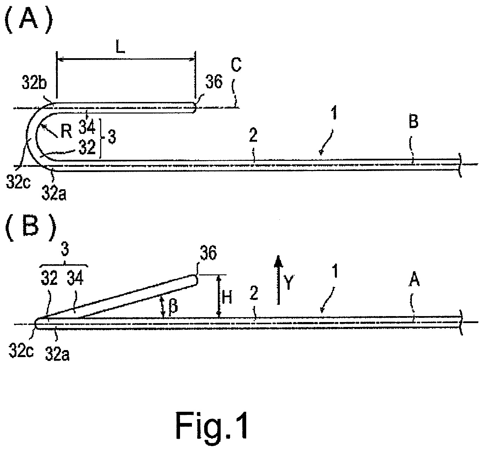

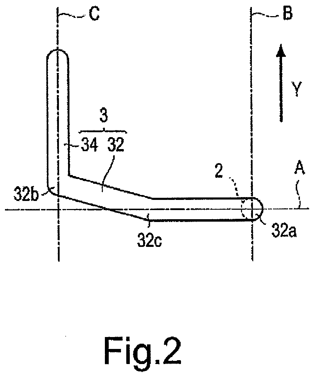

A guide wire for medical treatment 1 relating to an exemplified embodiment of the present invention includes, as shown in FIGS. 1 and FIG. 2, an approximately straight line shaped proximal section 2 and a distal section 3 positioned on the distal side of the proximal section 2 at the end portion on the side inserted inside a body cavity. Here, it is assumed that the distal direction indicates a direction toward which insertion is carried out inside the body cabity and the proximal direction indicates the opposite direction thereof. With respect to the distal section 3, a core metal composed of a metal, which will be mentioned later, forms a taper shape, but it is allowed even if the taper shape is not always employed.

The guide wire 1 is constituted such that a synthetic resin layer is coated on a core metal composed of a metal. As the material preferably used for the core metal, there can be cited a superelastic alloy such as an Ni-Ti alloy, a stainless steel alloy or the like in which it is allowed to be constituted by one kind of material or it is allowed to be constituted by joining two or more materials. Also, in order to obtain flexibility at the distal end, the core metal at the distal portion is diameter-reduced in a taper shape. As a resin material preferably used for the synthetic resin layer, there can be cited a polyurethane, a fluorine-based resin such as PTFE (polytetrafluoroethylene), a polyamide-based resin such as nylon, or polyolefin such as polyethylen and polypropylene. It is possible for those resins to be mixed with X-ray contrast fine particles such as of barium oxide, tungsten and the like. Also, by providing a coil of platinum, gold or the like for X-ray contrast at the distal portion of the core metal, it is also possible to employ such a constitution in which there can be carried out a work operation under X-ray fluoroscopy. It is preferable for the surface of the synthetic resin layer to be coated further with a hydrophilic lubricant coating composed of maleic anhydride or the like. Thus, insertion resistance inside a tube for medical treatment such as a catheter and inside a body cabity is reduced, and a smooth insertion therein becomes possible. It is preferable for the lubricant coating not to be coated on a portion which is not inserted into the body cabity inside.

It is preferable for the outer diameter of such a guide wire 1 to be around 0.1mm to 1.40mm normally, but it is not limited by these values. Further, the full length of the guide wire 1 is around 100cm to 450cm and preferably is around 120cm to 350cm, but it is not limited by these values.

The distal section 3 includes a curved portion 32 which is curved and a distal portion 34 continuous with the curved portion 32, and there is provided a most distal portion 36 at the most distal end of the distal portion 34. Caused by a fact that the curved portion 32 is curved, the guide wire 1 is constituted to form approximately J-shape such that the most distal portion 36 faces not toward the distal direction but toward the proximal direction in the original shape (shape in a state of being applied with no load at all).

The curved portion 32 includes a curve start point 32a which is a start point portion on the proximal side, a curve end point 32b which is an end point portion on the distal side and a curve midpoint 32c which is a midpoint between the curve start point 32a and the curve end point 32b. With respect to the curved portion 32, a portion from the curve start point 32a to the curve midpoint 32c is curved on a curve plane A which is an identical plane and on this curve plane A, there is positioned also the proximal section 2. Then, the region from the curve midpoint 32c to the curve end point 32b of the curved portion 32 is extended so as to back away gradually from the curve plane A along with approaching to the distal portion 34.

The distal portion 34 is extended so as to back away gradually from the curve plane A along with approaching to the most distal portion 36, and it is shaped such that the most distal portion 36 is positioned in a section in which the curved portion 32 is curved with respect to an orthogonal plane B which is a plane on the axis core of the proximal section 2 and which is orthogonal to the curve plane A shown in FIG. 2, that is, a section on the side in which the curved portion 32 is extended by making the orthogonal plane B as a boundary. In this exemplified embodiment, as shown in FIG. 1A, a projection line of the distal portion 34 onto the curve plane A is made to be approximately in parallel with the proximal section 2 and the projection line does not intersect with the proximal section 2. More specifically, the distal portion 34 does not intersect with the orthogonal plane B.

For a preferable embodiment of the curved portion 32, it is preferable for the radius of curvature R to be around 0.2mm to 20mm and more preferably, to be 0.5mm to 8mm.

Also, the inclination angle *β* of the region from the curve midpoint 32c to the curve end point 32b of the curved portion 32 and the distal portion 34 with respect to the curve plane A is made to be approximately constant, but it is allowed even if the angle is not always constant and it is preferable to satisfy a relation of -90degree < inclination angle *β* < 90degree. It should be noted that it is allowed even if the inclination angle of the curved portion 32 with respect to the curve plane A and the inclination angle of the distal portion 34 with respect to the curve plane A are different.

It is preferable for the height H of the most distal portion 36 from the curve plane A to satisfy a relation of 0mm < |H| < 20mm. It is preferable for the length L of the projection line of the distal portion 34 onto the curve plane A to be 0.5mm to 20mm.

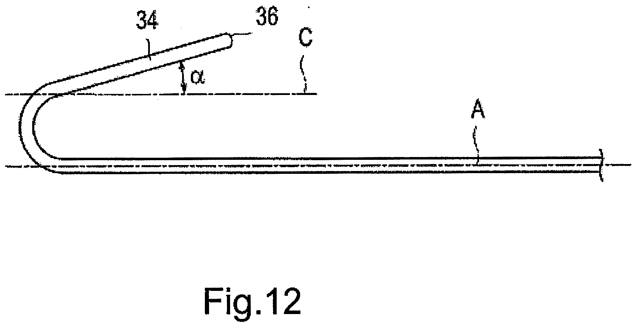

Also, bending angle *α* of the distal portion 34 with respect to the orthogonal plane B (see FIG. 12) is 0degree in this exemplified embodiment, but it is allowed to be set as 0degree < bending angle *α* < 90degree as shown in FIG. 12 in a range in which the projection line of the distal portion 34 to the curve plane A does not intersect with the proximal section 2, that is, in a range in which the distal portion 34 does not intersect with the orthogonal plane B

Next, it will be explained with respect to the operation of the guide wire for medical treatment 1 relating to the exemplified embodiment.

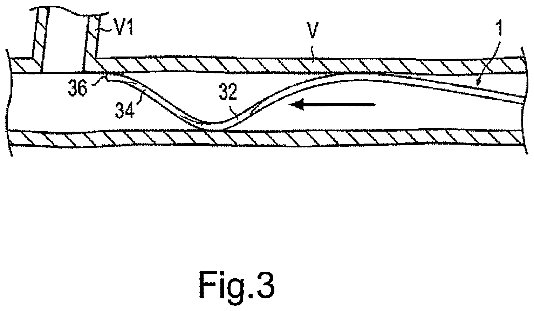

When an operator inserts the distal portion 34 of the guide wire 1 into the blood vessel V inside, the insertion is carried out by way of a tube shaped apparatus referred to as an inserter, but the insertion cannot be carried out by using the original shape directly for the reason that there is formed a three-dimensional shape, so that the operator extends the distal section 3 such that the most distal portion 36 thereof is directed toward the distal direction (insertion direction) and thereafter, inserts it into the inserter. At that time, the distal portion 34 is shaped by being extended so as to be apart from the curve plane A and therefore, when being extended, a spiral shape is formed in the vicinity of the curved portion 32. When insertion into the blood vessel V inside is carried out in this state, as shown in FIG. 3, a portion of the spiral shape contacts with the blood vessel wall, so that the shape as it is will be maintained.

Further, when the operator handles the proximal side of the guide wire 1 and continues to push ahead the distal end of the guide wire l toward a distal of the blood vessel V inside, it sometimes happens that the most distal portion 36 is to pass by an entrance of a branched blood vessel Vl. However, owing to a fact that the guide wire 1 relating to this exemplified embodiment has a spiral shape for the distal end thereof and the distal portion 34 becomes in a state of being inclined with respect to the direction of movement of the guide wire 1 while being wound around in a spiral circumferential direction, even if the most distal portion 36 contacts with the entrance the branched blood vessel Vl, the center axis of the distal portion 34 is deformed so as to be swung laterally such that the spiral pitch will be compushed, so that it is possible to distribute resistive force applied to the blood vessel wall which is a contact point of the entrance of the branched blood vessel V1. Thus, it is possible to decrease phenomena of the guide wire 1 going erroneously into the branched blood vessel Vl and also, it is possible to decrease confirmation operations by using contrast agents with respect to a patient, so that burden to the body with respect to the patient becomes less and also the expense for the contrast agent can be reduced.

Also, in this exemplified embodiment, the distal side from the curve midpoint 32c which is on the way of the curved portion 32 is extended in the original shape so as to back away gradually from the curve plane A, so that it is possible to heighten the restitutive force toward the spiral shape, it is easily possible to get a spiral shape by being extended and it is possible to further heighten an effect for distibuting the resistive force applied to the distal section 3.

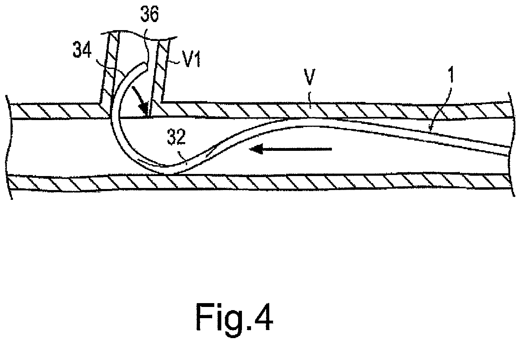

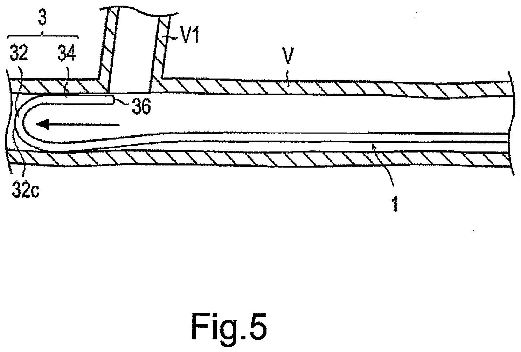

Also, as shown in FIG. 4, when the most distal portion 36 passes by the entrance of the branched blood vessel V1 and the operator pushes it further, the contact of the blood vessel wall and the distal portion 34 becomes a trigger, the distal portion 34 of the guide wire 1 is turned around and the most distal portion 36 is directed to the proximal direction, and as shown in FIG. 5, it becomes possible for the portion 36 to return to the original shape before the insertion into the blood vessel. Thus, the curved portion 32 of the guide wire 1 becomes the most distal end in the insertion direction and the guide wire becomes in a shape in which it is difficult for the most distal portion 36 to contact with the blood vessel wall even with respect to a forceful pushing-in of the operator and in which safety is heightened wherein damage of the blood vessel wall can be prevented. Also, it is constituted such that the curve midpoint 32c of the curved portion 32, which becomes the most distal end in the insertion direction, is positioned at the center of the blood vessel diameter, so that it becomes difficult for the curve midpoint 32c to contact with the blood vessel wall and it becomes possible to make the guide wire 1 go straight ahead smoothly inside the blood vessel V. In this manner, when the guide wire of the present invention is in a shape inserted into the blood vessel V inside (extended shape), property for preventing erroneous entering into the branched blood vessel and operability are improved, and when the distal section 3 is in an inverted shape, there can be further improved safety in a state of maintaining operability. More specifically, it is possible for the guide wire of the present invention to realize property for preventing erroneous entering into the branched blood vessel, operability and safety with excellent balance.

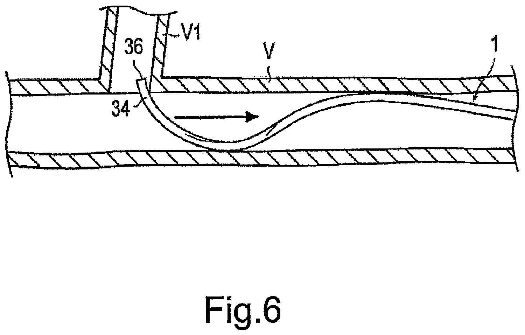

Also, in a case in which the distal section 3 of the guide wire 1 is inverted, the most distal portion 36 is faced to the proximal direction and thereafter, the guide wire 1 is desired to restore the extended shape again, as shown in FIG. 6, it is possible to restore the spiral shape again by hooking the most distal portion 36 of the guide wire 1 at the entrance of the branched blood vessel Vl and thereafter, by pulling back the guide wire 1 backward, and thereafter, it is possible to push ahead the guide wire 1 while handling the most distal portion 36 so as not pass by the entrance of the branched blood vessel Vl and while maintaining the spiral shape. At that time, the distal portion 34 including the most distal portion 36 is positioned at a section at which the curved portion 32 is curved with respect to the orthogonal plane B, so that it is possible to restore the spiral shape easily by pulling back the guide wire 1 backward. More specifically, in a case in which the most distal portion 36 is positioned on the opposite side of a section at which the curved portion 32 is curved with respect to the orthogonal plane B, the projection line of the distal portion 34 onto the curve plane A intersects with the proximal section 2, so that when the most distal portion 36 is hooked and the guide wire 1 is pulled back backward, it becomes easy for the distal portion 34 and the proximal section 2 to intersect with each other strongly, that is, it becomes easy to be deformed such that the radius of curvature R of the curved portion 32 becomes smaller, so that it becomes difficult to restore the spiral shape, but in this exemplified embodiment, the most distal portion 36 is positioned at a section at which the curved portion 32 is curved with respect to the orthogonal plane B and the distal portion 34 does not intersect with the orthogonal plane B, so that it is easy for the guide wire 1 to return to the spiral shape.

It should be noted that the present invention is not limited by the exemplified embodiment mentioned above and it is possible to revise variously within the scope of the claims.

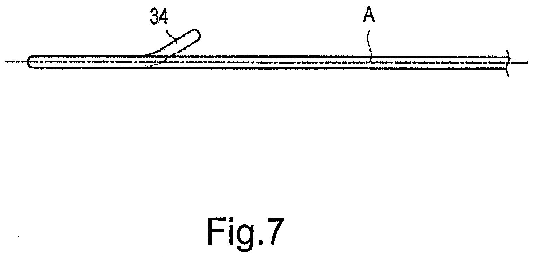

For example, the configuration of the distal portion 34 is not limited by the configuration shown in FIGS. 1 and FIG. 2. Consequently, for example, as shown in FIG. 7, it is allowed for only a portion on the distal side of the distal portion 34 to be extended so as to back away from the curve plane A gradually.

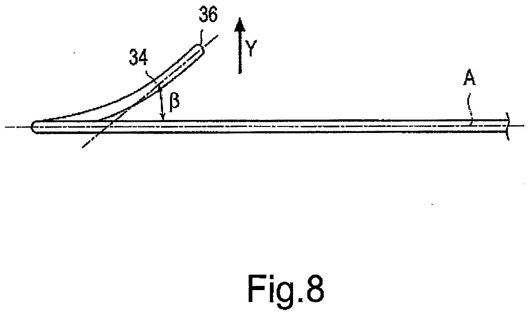

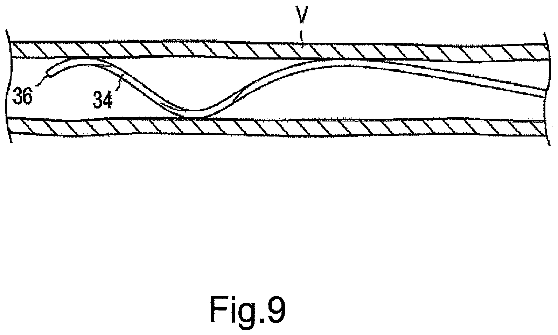

Also, it is also possible to employ a shape which is curved toward the Y direction 36 in which, as shown in FIG. 8, the inclination angle β of the distal portion 34 with respect to the curve plane A is made to become larger along with approaching to the most distal portion. By employing such a configuration, when being inserted into the blood vessel V inside, as shown in FIG. 9, it becomes possible for the most distal portion 36 so as not to contact with the blood vessel wall by making the region which is different from the most distal portion 36 of the distal portion 34 contact with the blood vessel wall and it is possible to heighten safety by reducing scratches or bumps with respect to the blood the vessel V caused by the most distal portion 36 of the guide wire 1. It should be noted that it is allowed not to employ a constitution in which the inclination angle *β* of the distal portion 34 with respect to the curve plane A becomes larger gradually along with approaching to the most distal portion 36, but to employ a constitution in which the angle becomes larger stepwise by being bent locally.

It should be noted that it is preferable for the degree of curve toward the Y direction of the distal portion 34 to be changed appropriately by the guide wire 1. As one example, in order to exert an effect for a subclavian artery having many branched blood vessels, the inner diameter of the average subclavian artery is to be around 6mm, so that when the guide wire l becomes in a spiral shape inside the 6mm inner diameter tube, it is preferable for the most distal portion 36 to be set so as not contact the inner wall of the tube and so as to be positioned approximately at the center of the inner diameter. By employing a constitution in which the most distal portion 36 is not contacted with the blood vessel wall and further, is positioned approximately at the center of the inner diameter, it is possible to repush erroneous insertion of the guide wire 1 into the branched blood vessel Vl more reliably and it is possible to push the guide wire 1 forward smoothly and also safely.

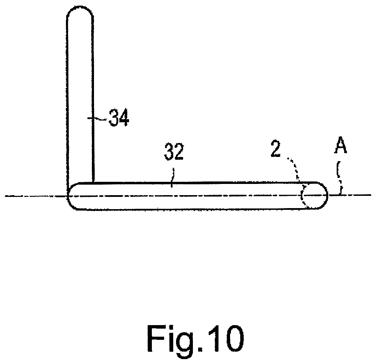

Also, the configuration of the curved portion 32 is not limited by the configuration shown in FIGS. 1 and FIG. 2 if at least a portion of the proximal side of the curved portion 32 is positioned on the curve plane A. Therefore, for example, as shown in FIG. 10, it is allowed for the whole curved portion 32 to be positioned on the curve plane A.

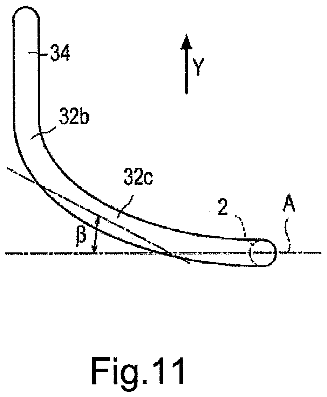

Also, as shown in FIG. 11, by employing a constitution in which the inclination angle *β* with respect to the curve plane A from the curve start point 32a to the curve end point 32b is made so as to become larger along with approaching to the distal portion 34, it is allowed to form a shape which is curved toward the Y direction backing away from the curve plane A. In this case, it happens that a tangent line at the curve start point 32a is to be positioned on the curve plane A. Also, it is allowed for the region curved toward the Y direction to be not only the whole of but also a portion of the curved portion 32 and in addition, it is allowed to be bent toward the Y direction locally.

Also, although it was mentioned before, as shown in FIG. 12, it is allowed for the most distal portion 36 of the distal portion 34 to be positioned on the side backing away from the orthogonal plane B other than on the plane C which is parallel with the orthogonal plane B and at which the curve end point 32b is positioned.

In addition, it is allowed for the distal section 3 to have such a constitution in which the rigidity thereof is heightened appropriately by being wound with a metal wire of coil or the like and it is also allowed to employ a constitution of a flat plate shape formed by pushing a metal core wire. Further, depending on different purposes, it is possible to appropriately change the position at which the metal wire of coil or the like is wound or the position at which the flat plate shape is formed by pushing the metal core wire.

In addition, it is possible for a guide wire relating to the present invention to be used for introducing a medical apparatus of a guide wire, a catheter or the like from a radial region, a brachial region and a femoral region to a target region of a chest region, a abdomen region or the like, and the scope of application thereof will be never limited by the sticking region or the target region.

### DESCRIPTION OF REFERENCE NUMERALS

- 1:: guide wire for medical treatment
- 2:: proximal section
- 3:: distal section
- 32:: curved portion
- 32a:: curve start point
- 32b:: curve end point
- 32c:: curve midpoint
- 34:: distal portion
- 36:: most distal portion
- A:: curve plane
- B:: orthogonal plane

## Claims

1. A guide wire (1) for medical treatment comprising a straight-line shaped proximal section (2) and a distal section (3) which is continuous with a distal side of the proximal section (2) and has a most distal portion (36) facing to the proximal direction, wherein
the distal section (3) includes a curved portion (32) having a curve start point (32a) continuous with the distal side of the proximal section (2) and a distal portion (34) continuous with a curve end point (32b) of the curved portion (32),
the proximal section (2) and at least a portion on the proximal side of the curved portion (32) including the curve start point (32a) are positioned on an identical curve plane (A), and
the distal portion (34) is extended toward a direction backing away from the curve plane (A),
**characterized in that**
the distal portion (34) includes the most distal portion (36) in a section of a direction to which the curved portion (32) is curved with respect to an orthogonal plane (B) on an axis core of the proximal section (2) orthogonal to the curve plane (A), and
the distal portion (34) does not intersect with the orthogonal plane (B).

2. The guide wire (1) for medical treatment according to claim 1, wherein with respect to the curved portion (32), at least a portion on the distal side thereof including the curve end point (32b) is extended toward a direction backing away from the curve plane (A).

3. The guide wire (1) for medical treatment according to claim 2, wherein a region from the curve start point (32a) to a curve midpoint (32c) of the curved portion (32) is curved on the curve plane (A).

4. The guide wire (1) for medical treatment according to claim 1 or 2, wherein with respect to the distal portion (34), an inclination angle (β) thereof with respect to the curve plane (A) changes toward a direction backing away from the curve plane (A).

5. The guide wire (1) for medical treatment according to any one of claims 1 to 4, wherein a projection line of the distal portion (34) including the most distal portion (36) onto the curve plane (A) is approximately parallel with the proximal section (2).

6. The guide wire (1) for medical treatment according to any one of claims 1 to 5, wherein the guide wire (1) is constituted such that a synthetic resin layer is coated on a core metal composed of a metal.

## Patentansprüche

1. Führungsdraht (1) zur medizinischen Behandlung, der ein geradlinig geformtes proximales Teilstück (2) und ein distales Teilstück (3) umfasst, welches mit einer distalen Seite des proximalen Teilstücks (2) zusammenhängt und einen distalsten Abschnitt (36) hat, der in die proximale Richtung weist, wobei
das distale Teilstück (3) einen gekrümmten Abschnitt (32) mit einem Kurvenstartpunkt (32a), der mit der distalen Seite des proximalen Teilstücks (2) zusammenhängt, und einen distalen Abschnitt (34) aufweist, der mit einem Kurvenendpunkt (32b) des gekrümmten Abschnitts (32) zusammenhängt,
das proximale Teilstück (2) und zumindest ein Abschnitt auf der proximalen Seite des gekrümmten Abschnitts (32), der den Kurvenstartpunkt (32a) aufweist, auf einer identischen Kurvenebene (A) positioniert sind und
der distale Abschnitt (34) zu einer Richtung hin verlängert ist, die von der Kurvenebene (A) zurückweicht,
**dadurch gekennzeichnet, dass**
der distale Abschnitt (34) den distalsten Abschnitt (36) in einem Teilstück einer Richtung aufweist, in der der gekrümmte Abschnitt (32) bezüglich einer orthogonalen Ebene (B) auf einem Achsenkern des proximalen Teilstücks (2), die zur Kurvenebene (A) orthogonal ist, gekrümmt ist, und
der distale Abschnitt (34) sich nicht mit der orthogonalen Ebene (B) schneidet.

2. Führungsdraht (1) zur medizinischen Behandlung nach Anspruch 1, wobei bezüglich des gekrümmten Abschnitts (32) zumindest ein Abschnitt auf der distalen Seite von ihm, der den Kurvenendpunkt (32b) aufweist, zu einer Richtung hin verlängert ist, die von der Kurvenebene (A) zurückweicht.

3. Führungsdraht (1) zur medizinischen Behandlung nach Anspruch 2, wobei ein Bereich von dem Kurvenstartpunkt (32a) zu einem Kurvenmittelpunkt (32c) des gekrümmten Abschnitts (32) auf der Kurvenebene (A) gekrümmt ist.

4. Führungsdraht (1) zur medizinischen Behandlung nach Anspruch 1 oder 2, wobei sich bezüglich des distalen Abschnitts (34) ein Neigungswinkel (β) von ihm bezüglich der Kurvenebene (A) zu einer Richtung hin ändert, die von der Kurvenebene (A) zurückweicht.

5. Führungsdraht (1) zur medizinischen Behandlung nach einem der Ansprüche 1 bis 4, wobei eine Projektionslinie des distalen Abschnitts (34), der den distalsten Abschnitt (36) aufweist, auf die Kurvenebene (A) ungefähr parallel zu dem proximalen Teilstück (2) ist.

6. Führungsdraht (1) zur medizinischen Behandlung nach einem der Ansprüche 1 bis 5, wobei der Führungsdraht (1) derart aufgebaut ist, dass eine Kunstharzschicht auf einem aus einem Metall bestehenden Kernmetall aufgeschichtet ist.

## Revendications

1. Fil-guide (1) pour un traitement médical comprenant une section proximale en forme de ligne droite (2) et une section distale (3) qui est continue avec un côté distal de la section proximale (2) et a la partie la plus distale (36) orientée dans la direction proximale, dans lequel :
la section distale (3) comprend une partie incurvée (32) ayant un point de départ de courbe (32a) continu avec le côté distal de la section proximale (2) et une partie distale (34) continue avec un point de fin de courbe (32b) de la partie incurvée (32),
la section proximale (2) et au moins une partie du côté proximal de la partie incurvée (32) comprenant le point de départ de courbe (32a), sont positionnées sur un plan de courbe identique (A), et
la partie distale (34) est étendue vers une direction s'éloignant du plan de courbe (A),
**caractérisé en ce que** :
la partie distale (34) comprend la partie la plus distale (36) dans une section d'une direction dans laquelle la partie incurvée (32) est incurvée par rapport à un plan orthogonal (B) sur un noyau axial de la section proximale (2) orthogonal par rapport au plan de courbe (A), et
la partie distale (34) ne coupe pas le plan orthogonal (B) .

2. Fil-guide (1) pour un traitement médical selon la revendication 1, dans lequel par rapport à la partie incurvée (32), au moins une partie sur son côté distal comprenant le point de fin de courbe (32b) est étendue vers une direction s'éloignant du plan de courbe (A).

3. Fil-guide (1) pour un traitement médical selon la revendication 2, dans lequel une région allant du point de départ de courbe (32a) à un point central de courbe (32c) de la partie incurvée (32) est incurvée sur le plan de courbe (A) .

4. Fil-guide (1) pour un traitement médical selon la revendication 1 ou 2, dans lequel, par rapport à la partie distale (34), son angle d'inclinaison (β) par rapport au plan de courbe (A) change vers une direction s'éloignant du plan de courbe (A).

5. Fil-guide (1) pour un traitement médical selon l'une quelconque des revendications 1 à 4, dans lequel une ligne de saillie de la partie distale (34) comprenant la partie la plus distale (36) sur le plan de courbe (A) est approximativement parallèle à la section proximale (2).

6. Fil-guide (1) pour un traitement médical selon l'une quelconque des revendications 1 à 5, dans lequel le fil-guide (1) est constitué de sorte qu'une couche de résine synthétique est appliquée sur un métal de noyau composé d'un métal.
